# EUROPEAN PATENT APPLICATION

(11) **EP 1 614 749 A1**
(43) Date of publication of application: **11.01.2006**
(21) Application number: 04723704.5
(22) Date of filing: 26.03.2004
(51) Int. Cl.: C12N 15/08, C12P 21/08

(54) **METHOD OF CONSTRUCTING HYBRIDOMA PRODUCING ANTIGEN-SPECIFIC ANTIBODY USING SINGLE ANTIGEN-SPECIFIC B LYMPHOCYTE AND METHOD OF PRODUCING MONOCLONAL ANTIBODY**

(30) Priority: 28.03.2003 JP 2003089725
(71) Applicant: Toyama New Industry Organizaion, Toyama-shi, Toyama 930-0866 (JP); Muraguchi, Atsushi, Toyama-shi, Toyama 930-0001 (JP); Kishi, Hiroyuki, Toyama-shi, Toyama 930-0884 (JP); Tamiya, Eiichi, Kanazawa-shi, Ishikawa 921-8105 (JP); Suzuki, Masayasu, Toyama-shi, Toyama 930-0962 (JP)
(72) Inventor: MURAGUCHI, Atsushi, Toyama-shi, Toyama 930-0001 (JP); KISHI, Hiroyuki, Toyama-shi, Toyama 930-0884 (JP); TAMIYA, Eiichi, Kanazawa-shi, Ishikawa 921-8105 (JP); SUZUKI, Masayasu, Toyama-shi, Toyama 930-0962 (JP); TOHBO, Kihachiro, Toyama-shi, Toyama 930-0042 (JP); UENO, Minoru, Toyama-shi, Toyama 930-0074 (JP); NAKAZATO, Hiroyoshi, Toyama-shi, Toyama 939-8058 (JP)
(74) Representative: Patentanwälte Ruff, Wilhelm, Beier, Dauster & Partner
(86) International application number: PCT/JP2004/004274
(87) International publication number: WO 2004/087911

(57) **Abstract**

A method for producing a monoclonal antibody and antigen-specific antibody-producing hybridomas using a single antigen-specific B lymphocyte is provided. The method for producing antigen-specific antibody-producing hybridomas comprises selecting a single B lymphocyte reacting specifically with a certain antigen, an "antigen-specific B lymphocyte", culturing the antigen-specific B lymphocyte selected, and fusing the cultured antigen-specific B lymphocyte with myeloma cells to obtain the hybridomas. The monoclonal antibody is produced with thus obtained hybridomas.

## Description

### Technical Field

The present invention relates to a method for manufacturing antigen-specific antibody-producing hybridomas employing a single antigen-specific B lymphocyte and to a method for producing monoclonal antibodies.

### Background Art

Conventionally, antigen-specific antibody-producing hybridomas are produced to manufacture monoclonal antibodies. In conventional methods of producing hybridomas, hybridoma clones producing antigen-specific antibodies are screened after preparing hybridomas. However, the preparation of hybridomas is not highly efficient. That is, not all B lymphocytes become hybridomas; only some of the B lymphocytes produced by cell fusion with myelomas become hybridomas. Further, producing hybridomas from spleen cells that have been stimulated with antigen does not exclusively yield antigen-specific antibody-producing hybridomas; most of the hybridomas obtained either produce unrelated antibodies or no antibody at all.

When attempting to find a hybridoma producing a target antibody by the conventional method, for example, spleen cells collected from an immunized mouse are fused with myelomas and plated on ten 96-well plates. It would be possible to plate even more cells, but there is a time constraint when a single person is conducting screening; unused cells are frozen and stored. By this method, hybridomas are grown in about 500 wells.

The hybridomas in the 500 wells do not all grow at the same speed; some grow quickly and others grow more slowly. Accordingly, it is impossible to simultaneously check the growth of all 500. First, a microscope is used to check whether cells in the wells are proliferating and whether a number of cells adequate to check for antibody have been reached. On that basis, the cell supernatants are collected from suitable wells and a check is made as to whether antigen-specific antibody is being produced. This checking for cells and checking of cell supernatants must be conducted rapidly because hybridomas grow rapidly and will exhaust the nutrients in the medium and die out if allowed to proliferate excessively. Accordingly, screening must be completed before desired hybridomas die out.

When a well is discovered in which a targeted hybridoma is growing, a hybridoma producing another antibody will often be found growing with the hybridoma producing the targeted antibody. Since hybridomas may expel their own chromosomes while growing, there are cases where a hybridoma that was producing an antibody loses the chromosome for the antibody and thus ceases to produce it. Such cells normally grow more rapidly than antibody-producing hybridomas. When left to themselves, most cultured cells become non-antibody producing cells. Accordingly, when a well in which a desired hybridoma is growing is discovered, the cells in that well must be plated one cell per well onto a 96-well plate (limiting dilution method) and screening conducted anew (secondary screening) for desired antibody-producing hybridomas. Once targeted hybridomas have been detected, it is necessary to rapidly complete secondary screening before the condition of the cells deteriorates.

As set forth above, since only a portion of the hybridomas prepared are screened and employed, it is difficult to obtain antigen-specific antibody-producing hybridomas of low frequency.

More specifically, in the case of human antigen-specific antibodies, there exists a method of transforming peripheral B lymphocytes with EB virus, culturing the transformants, and screening for cells producing antigen-specific antibodies (Lymphocyte Function Detection Methods (5^{th} Revised Edition), ed. by Junichi YATA and Michio FUJIWARA, Chugai Igakusha, 1994, *The Use of EB Virus Transformed Cells in Human Monoclonal Antibody Production,* Fumio MIZUNO, Toyoro OSATO, pp. 381-391). Since the frequency of the lymphocyte cell lines that can be established is low, the probability of obtaining an antigen-specific antibody-producing B lymphocyte cell line is extremely low. Further, about a month is required to establish a cell line. Still further, the B lymphocyte cell lines established produce small quantities of antibody. Murine hybridomas can be produced, but no system has been successfully devised for efficient human hybridomas.

Murine hybridomas can be produced. Conventionally, such hybridomas are produced by immunizing mice with antigen, removing the spleen or lymph nodes of the mice, preparing lymphocytes, fusing about 10⁸ of the lymphocytes prepared with about 10⁷ myeloma cells using polyethylene glycol or the application of a voltage, culturing the hybridomas in a selective medium such as HAT, screening the hybridomas that grow for those producing antigen-specific antibodies by ELISA, flow cytometry, or the like, and selecting the antigen-specific antibody-producing hybridomas (Lymphocyte Function Detection Methods (5^{th} Revised Edition), ed. by Junichi YATA and Michio FUJIWARA, Chugai Igakusha, 1994, *Monoclonal Antibody Preparation Methods Based on B Lymphocyte Hybridomas,* Hideo NARIUCHI, pp. 574-576; Monoclonal Antibodies in "Antibodies: A Laboratory Manual" by Ed Harlow and David Lane, Cold Spring Harbor Laboratory, Cold Spring Harbor, NY, pp. 139-244, 1988). Using this method, hybridomas can be grown in 300 to 400 wells, with several percent of the hybridomas that grow producing antigen-specific antibodies. This number varies with the antigen employed. When the frequency of the antigen-specific antibody-producing B lymphocyte is low, it is difficult to produce hybridomas by this method.

Accordingly, the present invention has for its object to provide a method for conveniently selecting a lymphocyte reacting specifically with a prescribed antigen, whether it be an antigen-specific lymphocyte of relatively high or low frequency, and producing antigen-specific antibody-producing hybridomas from the antigen-specific B lymphocyte selected.

A further object of the present invention is to provide a method of manufacturing monoclonal antibody from the antigen-specific antibody-producing hybridomas produced.

### Description of the Invention

The present invention relates to a method for producing antigen-specific antibody-producing hybridomas by selecting a single B lymphocyte reacting specifically with a certain antigen (referred to hereinafter as an "antigen-specific B lymphocyte"), culturing the antigen-specific B lymphocyte selected, and fusing the cultured antigen-specific B lymphocyte with myeloma cells.

In the above production method, the selected antigen-specific B lymphocyte can be cultured in the presence of anti-CD40 antibody, CD40 ligand (CD40L), and interleukins including interleukin 4 (IL-4),
in the presence of mitogen capable of inducing the proliferation of B lymphocytes (for example, LPS), or
in the presence of anti-CD40 antibody or CD40L; interleukins including IL-4; and mitogen (for example LPS).

The above single antigen-specific lymphocyte can be selected using a flow cytometer or microwell array chip.

The above single antigen-specific lymphocyte can be selected by adding antigen to the individual microwells of an antigen-specific lymphocyte detection-use microwell array chip having multiple microwells each containing a single lymphocyte specimen, detecting those lymphocytes reacting with the antigen, and removing the detected antigen-specific lymphocytes from the microwells.

The antigen-specific lymphocyte may be present at a frequency of less than or equal to 0.1 percent.

Further, the present invention relates to a method for manufacturing monoclonal antibody employing the hybridomas prepared by the above-described method of the present invention.

### Brief Description of the Drawings

Fig. 1 is a drawing descriptive of a method of detecting the change in concentration of Ca ions within the cell using a Ca-ion dependent fluorescent pigment.
Fig. 2 is a drawing descriptive of the distribution of cells on a microwell array chip, their stimulation with antigen, and their removal in a method employing fluorescent pigment.
Fig. 3 shows the results of ELISA detection of antibody to OVA in the serum of mice immunized with OVA.
Fig. 4 shows the results of ELISA detection of antibody in the culture supernatant of hybridomas producing OVA-specific antibody created by the method of the present invention.

### Best Mode of Implementing the Invention

The individual lymphocytes in the blood each react with different antigens. Accordingly, a B lymphocyte reacting specifically to an antigen is detected and hybridomas are prepared from this B lymphocyte in the present invention.

In conventional manufacturing of hybridomas, untreated spleen cells prepared from immunized mice are directly fused with myeloma cells to prepare hybridomas, the hybridomas are proliferated, and a check is made to determine whether the hybridomas produce antigen-specific antibody. By contrast, in the method of the present invention, B lymphocytes producing antigen-specific antibodies are detected and collected, and the antigen-specific B lymphocytes that have been collected are used to prepare hybridomas.

### (Selection of antigen-specific lymphocytes)

A single antigen-specific lymphocyte can be selected with a flow cytometer or a microwell array chip, for example.

A single antigen-specific lymphocyte can be selected using a flow cytometer by the usual method.

In methods employing microwell array chips, for example, antigen is added to each of the microwells of a microwell array chip for detecting antigen-specific lymphocytes having multiple microwells each containing a single lymphocyte specimen. Next, lymphocytes reacting with the antigen are detected and the antigen-specific lymphocytes that have been detected are removed from the microwells to obtain individual antigen-specific lymphocytes. This method will be described in greater detail.

### (The microwell array chip)

Any microwell array chip having multiple microwells each capable of containing a single lymphocyte specimen may be employed as the microwell array chip. Having each microwell contain a single lymphocyte specimen permits specification of antigen-specific lymphocytes at the cell level. That is, when employing such a microwell array chip, lymphocyte specimens reacting with antigen can be specified as single cells since a single lymphocyte specimen is contained in each microwell. As a result, an antigen-specific lymphocyte can be detected as a single cell and the single antigen-specific lymphocyte that has been detected can be removed and used to prepare a hybridoma.

However, cells other than lymphocytes may be contained with the specimen lymphocyte in a single microwell. This is because cells other than lymphocytes do not react with antigen and are not detected.

The shape and size of the microwell are not specifically limited. However, the shape of the microwell may be, for example, cylindrical. Other than cylindrical, it may be that of a rectangular parallelepiped or inverse conical. When the microwell is cylindrical in shape, for example, the diameter thereof can be from 5 to 100 micrometers, preferably 5 to 15 micrometers and the depth thereof can be 5 to 100 micrometers, preferably 5 to 30 micrometers.

The number of microwells present on each microwell array chip is not specifically limited. However, given that the frequency of antigen-specific lymphocytes is often only from 1 to at most 500 per 10⁵ lymphocytes, the number of microwells present per square centimeter can range from about 2,000 to 100,000, for example.

The lymphocyte specimen is contained in the microwell with culture medium. The following are examples of culture media suitable for use.
1. 137 mM NaCl, 2.7 mM KCI, 1.8 mM CaCl₂, 1 mM MgCl₂, 1 mg/mL glucose, 1 mg/mL BSA, 20 mM HEPES (pH 7.4).
2. RPMI 1640 culture medium containing 10 percent FCS (fetal calf serum).
3. RPMI 1640 culture medium 1 mg/mL BSA.
4. Dulbecco's MEM culture medium containing 10 percent FCS (fetal calf serum).
5. Dulbecco's MEM culture medium containing 1 mg/mL BSA.

The lymphocyte specimen may be derived from blood; for example, it may be a B lymphocyte or T lymphocyte. Further examples are lymphocytes derived from lymphoid tissues such as the tonsils (lymph nodes) and spleen, and lymphocytes infiltrating pathologically altered parts, such as cancer-infiltrating lymphocytes.

### (The method of detecting antigen-specific lymphocytes)

The method of detecting antigen-specific lymphocytes comprises the steps of adding antigen to each of the microwells in the above described microwell array chip, stimulating the cells, and detecting those cells reacting with the antigen.

The antigen may be added to the individual microwells in the following manner.
1. An antigen solution is supplied with a pipette in a manner covering the entire surface of the microwell array.
2. An antigen solution is supplied with an automatic spotter to each well.

The antigen that is detected by the method is not specifically limited; examples are proteins, peptides, DNA, RNA, lipids, sugar chains and the like. Further examples are bacteria, viruses, autoantigens, tumor antigens, allergens and the like.

The cells may be cultured by, for example, suspending the lymphocytes in culture medium, inserting them into microwells, and culturing them at room temperature or at 37°C in the air or in a CO₂ incubator.

The cells reacting with antigen are detected as follows:

When antigen binds to the antigen receptor (immunoglobulin) of a B lymphocyte, signal transduction first occurs within the cell, after which the cell proliferates and antibody production occurs. Accordingly, various methods may be employed to detect signal transduction within the cell, cell proliferation, and antibody production, thereby detecting cells reacting to antibody.

The detection of signal transduction within the cell to detect cells reacting with antigen, for example, can be conducted by detecting change in the concentration of Ca ions within the cell with Ca ion dependent fluorescent dyes.

When detecting change in the concentration of Ca ions within the cell, the fluorescent dye employed may be Fura-2, Fluo-3, or Fluo-4, and the detection device may be a fluorescence microscope or microarray scanner.

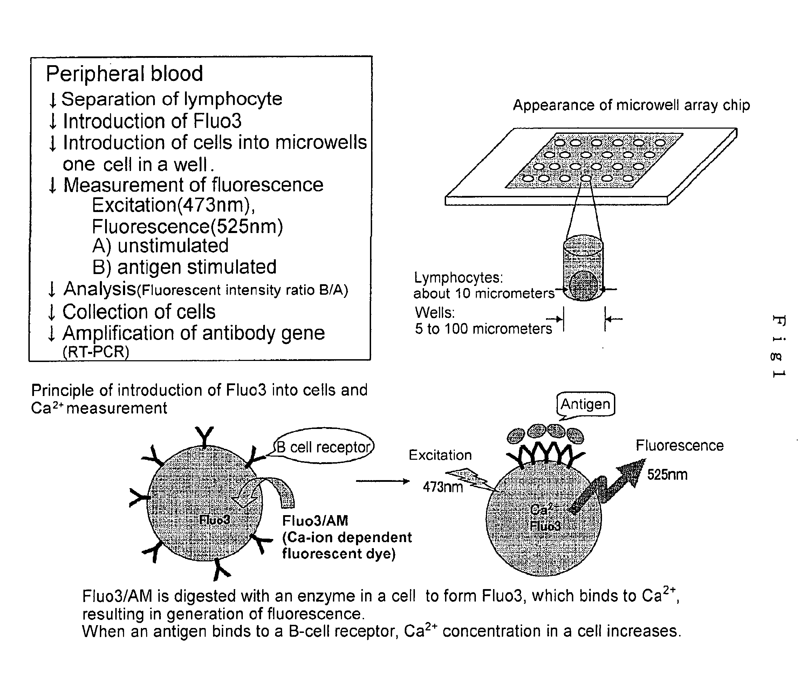

Specifically, as shown in Fig. 1, a Ca-ion dependent fluorescent dye such as Fura-2, Fluo-3 or Fluo-4 is introduced into the B lymphocyte. Next, the B lymphocyte is stimulated with antigen, causing the Ca ion concentration within the B lymphocyte to rise. As a result, Ca ions bind to the Ca ion dependent fluorescent dye, and the fluorescent intensity increases. Cells with low concentration of Ca ions are shown as bluish in color and cells with high concentration of Ca ions are shown as reddish color. This method permits the use of a microwell array chip to detect B lymphocytes (antigen specificity) in which the Ca ion concentration within the cells has increased due to stimulation with antigen.

In the detection of cell proliferation, cells reacting with antigen can be detected by measuring, for example, the number of cells by using a live cell-specific fluorescent dye. In this method, specifically, B lymphocytes are stimulated with antigen and cultured in a CO₂ incubator at 37°C for three days, causing the cells to proliferate. Once the cells have proliferated, fluorescein diacetate (FDA) or carboxy-fluorescein diacetate succinimidyl ester (CFSE) solution is added to the culture medium. These reagents pass through the membranes of living cells and are decomposed by esterase within the cells, producing a fluorescent dye that is incapable of passing throughout the membrane. The light emitted by this fluorescent dye is proportional to the number of cells, so the sum of the fluorescent intensity of the living cells within the well can be measured with a fluorescence microscope or microarray scanner to determine the number of living cells.

It is also possible to detect cells reacting with antigen by measuring the production of antibody. Antibody production can be detected by immunochemically measuring the antibody. Specifically, when B lymphocytes are stimulated with antigen, incubated in a CO₂ incubator for 37°C, and cultured for one week, they secrete antibody into the culture medium. Antigen-specific antibody that has been secreted into the culture medium can be detected by the ELISA method (enzyme-linked immunosorbent assay).

Alternatively, it is also possible to employ mitogen, lectin, antibody, cytokine, PMA, and Ca ionophore to detect signal transduction, cell proliferation, and antibody production.

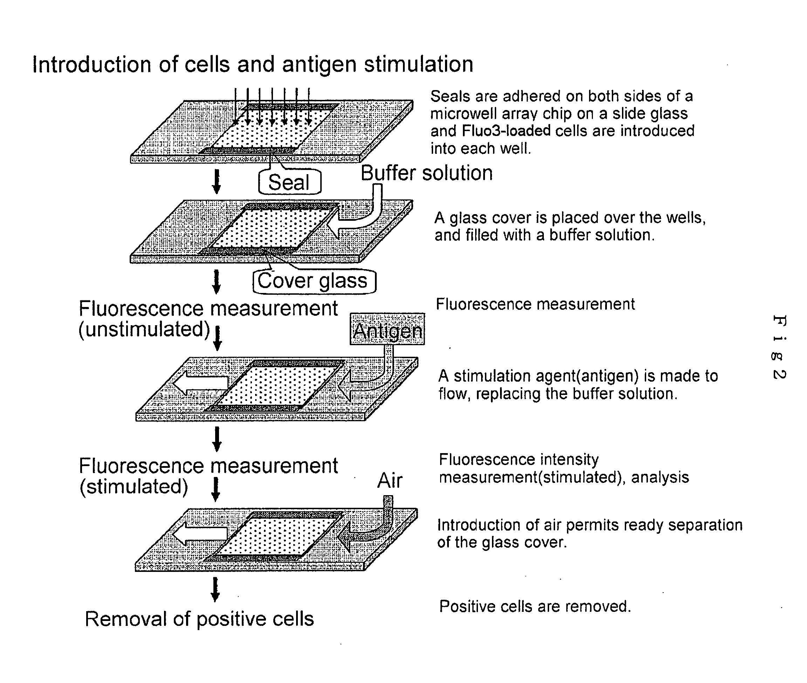

The introduction of cells onto the microwell array chip, their stimulation with antigen, and their removal will be described below based on Fig. 2.

### (1) Cell introduction

Single cells are introduced into each microwell.

The cells introduced into the microwells are prepared, for example, by separating the lymphocyte fraction from the spleen or lymph node of an immunized mouse, followed by further separation and purification of the B lymphocyte fraction.

Next, the cells are suspended in Fluo3/AM (2 micromole) solution, kept standing for 30 min. at room temperature, and washed with buffer solution to remove dye that has not been incorporated into the cells.

The cells are then introduced into the microwells. Both sides of the microwell array chip are sealed, a glass cover is placed thereover, and the space between is filled with buffer solution to prevent it from drying out.

### (2) Measuring fluorescence

First, the fluorescence of the unstimulated cells is measured and at the time, fluorescence intensity (A) is calculated. Next, an antigen solution is applied to flow between the glass slide and the glass cover, replacing the buffer solution, and the fluorescence of cells that have been stimulated by the antigen is measured. One or two minutes following stimulation, the fluorescent intensity (B) is measured. The cells in wells with a high ratio of fluorescence intensity (B/A) before and after stimulation are selected.

### (3) Removal of cells reacted to antibody stimulation

When air is introduced between the glass slide and the glass cover, the cover glass is readily removed. Cells that have reacted to antigen stimulation are selected based on the ratio (B/A) of fluorescence intensity of the cells after stimulation to the ratio of fluorescent intensity of the cells prior to stimulation and removed.

### (Preparation of hybridomas)

In the method of the present invention, a single antigen-specific B lymphocyte that has been selected is cultured and the antigen-specific B lymphocytes that are grown by culturing are fused with myeloma cells to prepare hybridomas. In reality, it is difficult to prepare a hybridoma from a single B lymphocyte. Accordingly, the single antigen-specific B lymphocyte is cultured and proliferated. The antigen-specific B lymphocyte can be cultured in the presence of anti-CD40 antibody or CD40 ligand (CD40L) and interleukins including interleukin 4 (IL-4). In this method, the antigen-specific B lymphocyte is caused to grow by stimulation by CD40 and IL-4. The proliferation method itself is known (Banchereau J., de Paoli P., Valle A., Garcia E., Rousset F. Long-term human B cell lines dependent on interleukin-4 and antibody to CD40. Science 251: 70-72, 1991). However, there is no previous example of the use of this method to prepare hybridomas.

B lymphocytes may be cultured in the presence of a mitogen (for example, lipopolysaccharide (LPS)) capable of inducing B lymphocytes to proliferate. They may also be cultured in the presence of anti-CD40 antibody or CD40L, interleukins including IL-4, and LPS. In addition to LPS, examples of mitogens inducing B cell proliferation are phorbol 12-myristate 13-acetate (PMA), Ca ionophores, and anti-immunoglobulin antibody.

The preparation of hybridomas using B lymphocytes stimulated with a mitogen such as LPS is known (for example, see: (1) Van Snick J.L., Coulie P., Monoclonal anti-IgG autoantibodies derived from lipopolysaccharide-activated spleen cells of 129/Sv mice. Journal of Experimental Medicine, 155:219-230, 1982; (2) Lange M., Le Guern C., Cazenave P.A., Covalent coupling of antigens to chemically activated lipopolysaccharide: a tool for in vivo and in vitro specific B cell stimulation. Journal of Immunological Methods, 63:123-131, 1983). However, there is no example of the preparation of hybridomas by inducing the proliferation of a single antigen-specific B lymphocyte.

Specifically, the antigen-specific B lymphocyte can be cultured in the following manner.
1. Antigen-specific B lymphocytes recovered from microwells are transferred to a 96-well plate that has been precoated with anti-CD40 antibody or CD40 ligand (CD40L). The wells are preloaded with a cell culture solution (RPMI 1640 medium containing 10 percent FCS) containing 200 microliters of interleukin 4 (IL-4).
2. The 96-well plate is transferred to a CO₂ incubator (5 percent CO₂) and the cells are cultured for one week to 10 days at 37°C.
3. The B lymphocytes grown with the CD40 and IL-4 signals are recovered from the wells and fused with myeloma cells by known methods to prepare hybridomas.
4. When adding CD40 and IL-4, instead of employing soluble anti-CD40 antibody or CD40L, CD40L and IL-4 genes can be introduced into cells, adherent cells producing CD40L and IL-4 can be cultured in advance in the wells of the 96-well plate, and the antigen-specific B lymphocytes that are recovered can be cultured over these cells.
5. In addition to IL-4, it is also possible to add IL-2, IL-5, IL-6, and the like.

In the method for preparing hybridomas of the present invention, lymphocytes prepared from mice immunized with antigen are added to a microwell array chip and stimulated with antigen to identify a single antigen-specific B lymphocyte. The single antigen-specific B lymphocyte that is identified is induced to proliferate and the lymphocytes obtained are used to prepare hybridomas. Accordingly, most of the antibody-producing hybridomas that are grown can be anticipated to produce antigen-specific antibodies. Since antigen-specific B lymphocytes of low frequency can be detected in microwell array chips, it becomes possible to produce hybridomas producing antibody to antigen that were previously difficult to prepare.

Although conventional hybridoma preparation methods require manpower and time, the use of microarray chips permits more rapid detection of antigen-specific B lymphocytes, saving both manpower and time.

### Examples

The present invention is described in greater detail below through examples.

### 1. Separation of B lymphocytes

The lymphocyte fraction was separated from the spleen and lymph node of an immunized mouse. The B lymphocyte fraction was then further separated and purified from the lymphocyte fraction using an AutoMACS (Miltenyi Biotec, Bergisch Gladbach, Germany).

### 2. Introduction of Fluo3 into cells (see Fig. 1)

2 x 10⁶ cells of B lymphocytes were suspended in 2 micromoles of Fluo3/AM (Dojin, Kumamoto)/loading buffer (137 mM NaCl, 2.7 mM KCI, 1.8 mM CaCl₂, 1 mM MgCl₂, 1 mg/mL glucose, 1 mg/mL BSA, and 20 mM HEPES (pH 7.4)) and incubated for 30 min at room temperature. The cells were washed with loading buffer to remove the Fluo3/AM that had not been incorporated into the cells. Subsequently, the cells were suspended in RPMI 1640/10 percent FCS solution.

### 3. Microwell array chip (see Fig. 2)

The microwell array chip was made of poly(dimethylsiloxane) (PDMS) or silicon and had microwells of 10 micrometers in diameter and 20 micrometers in depth arranged horizontally and vertically at a spacing of 30 micrometers (the center-to-center distance of the microwells was 40 micrometers) on a 2 x 2 cm chip. Seals with a thickness of 1 mm, a width of about 1 mm, and a length of 2 cm were adhered to both sides of the chip.

### 4. The microarray scanner

The device employed was basically a Hitachi Software Engineering (K.K.) (Yokohamashi) Microarray Scanner (CRBIO IIe) with the following change: one (Cy5-use, 635 nm) of the built-in lasers (Cy3-use, 532 nm; Cy5-use, 635 nm) was replaced with a 473 nm laser.

### 5. Detection of activated B lymphocytes using a microwell array chip (see Fig. 2)

The above-described cell suspension was added to the above-described microwell array chip and kept standing for five minutes. Cells that had not entered microwells were washed away with RPMI 1640/10 percent FCS. The diameter of the lymphocytes was about 10 micrometers. Since the diameter of the microwells employed was 10 micrometers, a single lymphocyte entered each microwell. A glass cover was placed over the above-described seal and the space between the chip and the glass cover was filled with RPMI 1640/10 percent FCS solution. The microwell array chip was inserted into a microarray scanner and scanned at a resolution of 2.5 micrometers. The data were stored (data A: fluorescence prior to antigen stimulation).

Next, the RPMI 1640/10 percent FCS solution between the chip and the glass cover was removed and the space was filled with antigen (10 micrograms/mL) dissolved in RPMI 1640/10 percent FCS solution. One minute after the antigen was added, the microwell array chip was inserted into the microarray scanner and scanned at a resolution of 2.5 micrometers. The data were stored (data B: fluorescence following antigen stimulation).

The ratio (B/A) of fluorescent intensity before and after stimulation was calculated, and wells with high ratios were specified. Antigen-specific B lymphocytes were present in these wells.

### 6. Separating out cells from the microwells

The cells were separated out under a microscope using a glass capillary with a micromanipulator.

### 7. Method of preparing hybridomas

1) To the wells of a 96-well plate are added 10 micrograms/mL of anti-CD40 antibody (eBioscience) and the antibody is incubated overnight to coat the wells with anti-CD40 antibody. Cells that have been isolated are added to medium (RPMI 1640 containing 10 percent FCS, 100 U/mL recombinant IL-4 (eBioscience)) that has been previously added to the wells of the 96-well plate, and the cells are cultured for about one week to 10 days in the presence of 5 percent CO₂ at 37°C.
2) The cells are removed from the wells and transferred to Eppendorf tubes. Approximately 200 mouse myeloma cells (X63.ag8.653) are added and the mixture is centrifuged for two minutes at 2,000 rpm.
3) The supernatant is removed, taking care not to disturb the precipitating residue of the cells. One mL of RPMI 1640 medium (not containing FCS) is added, the cells are suspended, and the mixture is centrifuged for two minutes at 2,000 rpm.
4) The supernatant is removed, taking care not to disturb the precipitating residue of the cells, and further centrifuged for 10 seconds at 2,000 rpm.
5) The supernatant is completely removed, taking care not to disturb the precipitating residue of the cells.
6) A 20 microliter quantity of 30 percent polyethylene glycol (Sigma) is added and the cells are lightly stirred.
7) The mixture is centrifuged for five minutes at 2,000 rpm. A 500 microliter quantity of RPMI 1640 medium (not containing FCS) is added and the cells are suspended. Next, 500 microliters of RPMI 1640 medium containing 20 percent FCS is added and the cells are suspended.
8) Following centrifugation for five minutes at 2,000 rpm, the supernatant is removed and the cells are suspended in 1 mL of HAT selective medium containing 20 percent FCS. Another 20 mL of HAT selective medium containing 20 percent FCS is added, and the mixture is placed 2 mL per well on a 24-well plate.
   HAT selective medium: RPMI 1640, 1 x 10⁻⁴ M hypoxanthine, 4 x 10⁻⁷ M aminopterin, 1.6 x 10⁻⁵ M thymidine.
9) The cells are cultured for 7 to 8 days in the presence of 5 percent CO₂ at 37°C.
10) Proliferation of the hybridomas is observed under an optical microscope and the presence of antibody in the culture supernatant is detected by ELISA or the like.

### 8. Method of preparing hybridomas (Alternative)

1) B lymphocytes are cultured (37°C, 5 percent CO₂) for one week in RPMI 1640 medium (200 microliters/well) containing 10 percent FCS, and the culture supernatant (5 percent v/v) of human T cells stimulated with 50,000 EL4 cells exposed to radiation (50 Gy), 500 U/mL of human IL-2 and 5 ng/mL of PMA and PWM.
2) A 100 microliter quantity of the culture supernatant is removed, 10,000 adherent cells expressing CD40 ligand that have been exposed to radiation (50 Gy) and 5 ng/mL of human IL-4 are added, and the mixture is cultured (37°C, 5 percent CO₂) for one week in RPMI 1640 medium (200 microliters/well) containing 10 percent of FCS and the culture supernatant (5 percent v/v) of human T cells stimulated with 500 U/mL of human IL-2 and 5 ng/mL of PMA and PWM.
3) A 100 microliter quantity of the culture supernatant is removed, 10,000 adherent cells expressing CD40 ligand that have been exposed to radiation (50 Gy) are added, and the mixture is cultured (37°C, 5 percent CO₂) for one week in RPMI 1640 medium (200 microliters/well) containing 10 percent of FCS and the culture supernatant (5 percent v/v) of human T cells stimulated with 5 ng/mL of IL-4, 500 U/mL of human IL-2, and 5 ng/mL of PMA and PWM.

Subsequently, the operations of 2) to 10) in the above-described "7. Method of preparing hybridomas" is repeated and hybridomas are detected.

### (Preparation of anti-OVA antibody-producing hybridomas)

An example of the preparation of anti-OVA antibody-producing hybridomas is given below.

### Immunization

A mixture of 10 micrograms of ovalbumin (OVA) mixed with Freund's complete adjuvant was injected subcutaneously into BALB/c mice. Two weeks later, 10 micrograms of OVA mixed with Freund's incomplete adjuvant was injected subcutaneously into the same mice. Two weeks later, 10 micrograms of OVA mixed with Freund's incomplete adjuvant was injected subcutaneously into the same mice. Subsequently, the mouse serum was collected and the production of antibodies to OVA was checked by ELISA by the following method.

The anti-OVA antibody in the serum was detected by the following method.

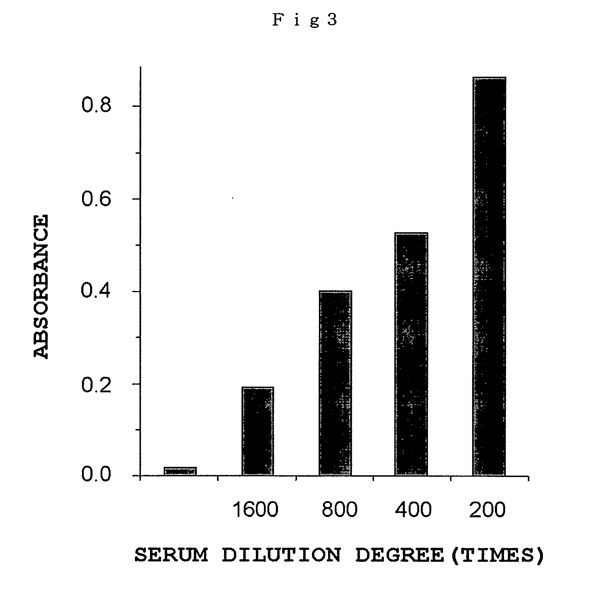

A 96-well plate was coated with OVA protein and blocking was made in a manner preventing nonspecific binding. Diluted serum obtained from the immunized mice was added to each well and reacted for two hours at room temperature. The mixture was then cleaned, alkaline phosphatase-labeled anti-mouse immunoglobulin was added, and the mixture was reacted for another two hours at room temperature. Following washing, alkaline phosphatase substrate was added, the mixture was reacted for 15 minutes at room temperature, and absorbance at 414 nm was measured. The results are given in Fig. 3.

### Loading of fluorescent pigment on the cells

Two weeks later, 10 micrograms of OVA dissolved in PBS were injected into the abdominal cavity. Four days later, the spleen was removed and spleen cells were prepared. The spleen cells were suspended to a cell density of 10⁷/mL in buffer solution A (20 mM of HEPES, pH 7.4, 137 mM of NaCl, 2.7 mM of KCl, 1.8 mM of CaCl₂, 1 mM of MgCl₂, 1 mg/mL of glucose, and 1 mg/mL of BSA) containing 1 microM Fluo-4 AM, 1 microM CellTracker Orange, and 0.02 percent Pluronic F-127, and incubated at room temperature for 30 minutes. Subsequently, the cells were washed with buffer solution A, the Fluo-4 and CellTracker Orange that had not been incorporated into the cells was removed, and the cells were suspended to 10⁵/microliters in RPMI 1640 solution (buffer solution B) containing 10 percent FCS.

### Detection of OVA-specific B lymphocytes

Spleen cells (10⁵/microliter) loaded with fluorescent pigment were added to a microwell array chip (made of silicon, 10 micrometer well diameter, approximately 15 micrometer well depth, 20 micrometer well pitch, about 240,000 wells) and the surplus cells that had not entered the wells were removed. The chip was covered with a glass cover, inserted into a CRBIO IIe-FITC made by Hitachi Software Engineering (K.K.), excited at a wavelength of 473 nm, and scanned for Flou-4 fluorescence at a wavelength of 535, and then excited at a wavelength of 535 nm, and scanned for CellTracker Orange fluorescence at a wavelength of 585. Next, the chip was removed from the scanner, the buffer solution B between the chip and the glass cover was removed, and OVA dissolved in buffer solution B (100 micrograms/mL) was added therebetween. The chip was inserted into the scanner and scanned at a resolution of 2.5 micrometers one minute after the addition of the OVA. A ratio of Fluo-4 fluorescence before and after stimulation was computed, and the addresses of cells exhibiting a fivefold to tenfold increase in fluorescence were specified.

### Recovery and culturing of OVA-specific B lymphocytes

Buffer solution B that had entered between the chip and the glass cover was removed and the glass cover was taken off. Subsequently, buffer solution B was added to the chip to prevent drying. The chip was placed on a fluorescent microscope and observed while using a micromanipulator to recover the OVA-specific B lymphocytes that had been identified with the scanner.

The B lymphocytes recovered were cultured (37°C, 5 percent CO₂) for one week in RPMI 1640 medium (200 microliters/well) containing 10 percent FCS and culture supernatant (5 percent v/v) of T cells stimulated with 50,000 EL4 cells exposed to radiation (50 Gy), 500 U/mL of human IL-2, and 5 ng/mL of PMA and PWM. Next, 100 microliters of the culture supernatant was removed, 10,000 CD40 ligand-expressing adherent cells that had been exposed to radiation (50 Gy) and 5 ng/mL of human IL-4 were added, and the cells were cultivated (37°C, 5 percent CO₂) for one week in RPMI 1640 medium (200 microliters/well) containing 10 percent FCS and the culture supernatant (5 percent v/v) of human T cells stimulated with 500 U/mL of human IL-2, and 5 ng/mL of PMA and PWM. Then, 100 microliters of the culture supernatant was removed, 10,000 CD40 ligand-expressing adherent cells that had been exposed to radiation (50 Gy) were added, and the cells were cultivated (37°C, 5 percent CO₂) for one week in RPMI 1640 medium (200 microliters/well) containing 10 percent FCS and the culture supernatant (5 percent v/v) of human T cells stimulated with 5 ng/mL of human IL-4, 500 U/mL of human IL-2, and 5 ng/mL of PMA and PWM.

### Preparation of hybridomas

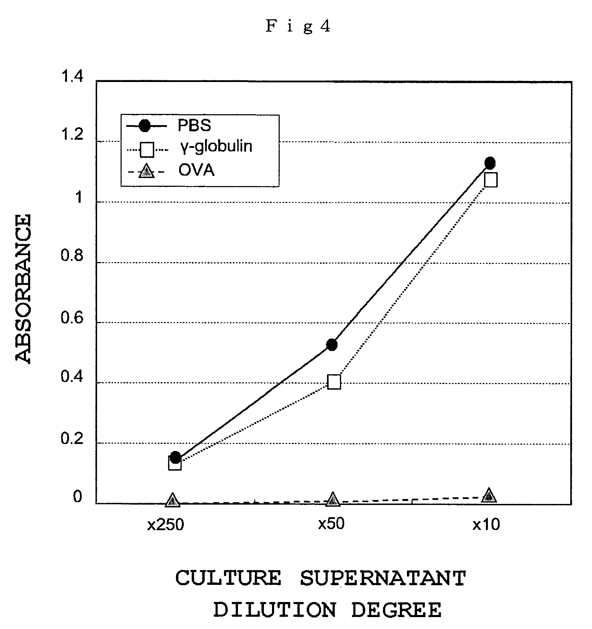

Proliferation of the B lymphocytes was observed with an optical microscope, cells were recovered from wells with proliferation, and the cells were transferred to Eppendorf tubes. To the tubes were added about 200 murine myeloma cells (X63.Ag8. 653) and the mixture was centrifuged for 2 minutes at 2,000 rpm. The supernatant was removed, taking care not to disturb the precipitating residue of the cells. One mL of RPMI 1640 medium (not containing FCS) was added, the cells were suspended, and the mixture was centrifuged for two minutes at 2,000 rpm. The supernatant was removed, taking care not to disturb the precipitating residue of the cells, centrifugation was conducted for another 10 seconds at 2,000 rpm, and the supernatant was completely removed, taking care not to disturb the precipitating residue of the cells. A 20 microliter quantity of 30 percent polyethylene glycol (Sigma) was added, the cells were lightly stirred, and centrifugation was conducted for five minutes at 2,000 rpm. A 500 microliter quantity of RPMI 1640 medium (not containing FCS) was added and the cells were suspended. Next, 500 microliters of RPMI 1640 medium containing 20 percent FCS was added and the cells were suspended. Following centrifugation for 5 minutes at 2,000 rpm, the supernatant was removed and the cells were suspended in 1 mL of HAT selective medium (RPMI 1640, 1 x 10⁻⁴ M hypoxanthine, 4 x 10⁻⁷ M aminopterin, and 1.6 x 10⁻⁵ M thymidine) containing 20 percent FCS. A 20 mL quantity of HAT selective medium containing 20 percent FCS was added, the mixture was added 2 mL per well to a 24-well plate, and the cells were cultivated for 7 to 10 days in the presence of 5 percent CO₂ at 37°C. The growth of hybridomas was observed under an optical microscope and anti-OVA antibody in the culture supernatant was detected by ELISA, described below. Fig. 4 gives the results of ELISA for anti-OVA antibody detected in the culture supernatant of the hybridomas. The absorbance changed with the dilution ratio of the culture supernatant. Since it was possible to specifically block binding of antibody to the OVA on the bottom of the well by adding soluble OVA to the culture supernatant, it was determined that anti-OVA antibody was produced in the culture supernatant.

The anti-OVA antibody in the hybridoma supernatant was detected as follows.

A 96-well plate was coated with OVA protein and blocking was conducted to prevent nonspecific binding. Diluted hybridoma culture supernatant (PBS) was added to each of the wells. Simultaneously with the addition of hybridoma culture supernatant, 2 micrograms/mL of OVA or *gamma-*globulin was added to other wells to check the specificity of the ELISA reaction. After reacting for two hours at room temperature, the mixture was washed, alkaline phosphatase-labeled anti-mouse immunoglobulin was added, and the mixture was reacted for another two hours at room temperature. After washing, alkaline phosphatase substrate was added, the mixture was reacted for 15 minutes at room temperature, and absorbance was measured at 414 nm. The results are given in Fig. 4.

### Industrial Applicability

According to the present invention, by selecting and collecting antigen-specific antibody-producing B lymphocytes and using them to prepare hybridomas, the labor required to screen antigen-specific antibody-producing hybridomas is greatly reduced and the efficiency with which hybridomas are prepared is greatly improved over conventional hybridoma preparation methods. Specifically, the following are achieved.
(a) Since the number of cells being handled decreases, the number of wells on the plate during plating is smaller. Since a small number of cells are handled, the time required for screening decreases and the burden on personnel performing the screening is reduced.
(b) When the hybridomas proliferate, although hybridomas not producing antigen-specific antibodies also proliferate, the frequency of such negative cells is lower because of the initial selection and concentration of antigen-specific antibody-producing B lymphocytes.
(c) The reliable preparation of hybridomas by the detection, collection, and proliferation of low frequency antigen-specific antibody-producing B cells using microwell array chips permits the preparation of hybridomas derived from antigen-specific antibody-producing B cells of low frequency the preparation of which has previously been impossible.

## Claims

1. A method for producing antigen-specific antibody-producing hybridomas by selecting a single B lymphocyte reacting specifically with a certain antigen, referred to hereinafter as an "antigen-specific B lymphocyte", culturing the antigen-specific B lymphocyte selected, and fusing the cultured antigen-specific B lymphocyte with myeloma cells.

2. The method of claim 1, wherein the selected antigen-specific B lymphocyte is cultured in the presence of anti-CD40 antibody, CD40 ligand (CD40L), and interleukins including interleukin 4 (IL-4).

3. The method of claim 1, wherein the selected antigen-specific B lymphocyte is cultured in the presence of mitogen capable of inducing the proliferation of B lymphocytes.

4. The method of claim 1, wherein the selected antigen-specific B lymphocyte is cultured in the presence of anti-CD40 antibody or CD40L; interleukins including IL-4; and mitogen.

5. The method of any of claims 1 to 4, wherein the single antigen-specific lymphocyte is selected using a flow cytometer or microwell array chip.

6. The method of any of claims 1 to 4, wherein the single antigen-specific lymphocyte is selected by adding antigen to the individual microwells of an antigen-specific lymphocyte detection-use microwell array chip having multiple microwells each containing a single lymphocyte specimen, detecting those lymphocytes reacting with the antigen, and removing the detected antigen-specific lymphocytes from the microwells.

7. The method of any of claims 1 to 6, wherein the antigen-specific lymphocyte is present at a frequency of less than or equal to 0.1 percent.

8. A method for manufacturing monoclonal antibody employing the hybridomas prepared by the method of any of claims 1 to 7.
